Europäisches Patentamt

European Patent Office

Office européen des brevets

(19)

(11) **EP 1 379 262 B1**

(12) **FASCICULE DE BREVET EUROPEEN**

(45) Date de publication et mention
de la délivrance du brevet:
**06.10.2004 Bulletin 2004/41**

(21) Numéro de dépôt: **02761922.0**

(22) Date de dépôt: **09.04.2002**

(51) Int Cl.$^7$: **A61K 35/78**, A61P 9/00,
A61P 25/00

(86) Numéro de dépôt international:
**PCT/FR2002/001219**

(87) Numéro de publication internationale:
**WO 2002/083158 (24.10.2002 Gazette 2002/43)**

(54) **PROCEDE DE PREPARATION D'UN EXTRAIT DE FEUILLES DE GINKGO BILOBA HAUTEMENT ENRICHI EN PRINCIPES ACTIFS**

**VERFAHREN ZUR HERSTELLUNG EINES AN WIRKSTOFFEN HOCHANGEREICHERTEN EXTRAKTES DER BLÄTTER VON GINKGO BILOBA**

**METHOD FOR PREPARING AN EXTRACT OF GINKGO BILOBA LEAVES HIGHLY ENRICHED IN ACTIVE PRINCIPLES**

(84) Etats contractants désignés:
**AT BE CH CY DE DK ES FI FR GB GR IE IT LI LU MC NL PT SE TR**

(30) Priorité: **10.04.2001 FR 0104942**

(43) Date de publication de la demande:
**14.01.2004 Bulletin 2004/03**

(73) Titulaire: **SOCIETE DE CONSEILS DE RECHERCHES ET D'APPLICATIONS SCIENTIFIQUES S.A. (S.C.R.A.S.) 75016 Paris (FR)**

(72) Inventeur: **TENG, Beng-Poon F-91190 Gif-sur-Yvette (FR)**

(74) Mandataire: **Bourgouin, André Beaufour Ipsen - S.C.R.A.S., 24, rue Erlanger 75781 Paris Cedex 16 (FR)**

(56) Documents cités:
- **DATABASE WPI Section Ch, Week 200143 Derwent Publications Ltd., London, GB; Class B05, AN 2001-398915 XP002184929 & CN 1 287 121 A (UNIV TIANJIN), 14 mars 2001 (2001-03-14)**
- **DATABASE WPI Section Ch, Week 199602 Derwent Publications Ltd., London, GB; Class B03, AN 1996-018388 XP002184930 & KR 9 402 796 B (SUN KYONG IND LTD), 2 avril 1994 (1994-04-02)**
- **DATABASE BIOSIS [en ligne] BIOSCIENCES INFORMATION SERVICE, PHILADELPHIA, PA, US; mars 2000 (2000-03) KOCH E ET AL: "Evidence for immunotoxic effects of crude Ginkgo biloba L. leaf extracts using the popliteal lymph node assay in the mouse." Database accession no. PREV200000180755 XP002184928 & INTERNATIONAL JOURNAL OF IMMUNOPHARMACOLOGY, vol. 22, no. 3, mars 2000 (2000-03), pages 229-236, ISSN: 0192-0561**

EP 1 379 262 B1

**Description**

L'invention concerne un procédé de préparation d'un extrait de feuilles de *Ginkgo biloba* hautement enrichi en principes actifs.

Certaines préparations phytopharmaceutiques standardisées à base d'extraits de feuilles de *Ginkgo biloba* sont actuellement utilisées pour traiter des désordres vasculaires cérébraux et périphériques, notamment en Europe, aux Etats-Unis et en Extrême-Orient.

L'extrait de *Ginkgo biloba* le plus couramment utilisé à l'heure actuelle (EGb 761®) contient 24 % de flavone-glycosides, 3 % de ginkgolides A, B, C et J au total et 3 % de bilobalide (cf. K. Drieu, *La Presse Médicale* (1986), **15**, 1455-1457). La dose journalière recommandée pour un patient est de 120 milligrammes.

Le brevet US 5,399,348 décrit par exemple une méthode pour préparer un extrait de *Ginkgo biloba* avec une composition moyenne de 24 % de flavone-glycosides, 3 % de ginkgolides A, B, C et J et 3 % de bilobalide. Cette méthode comprend les étapes suivantes :

i. extraction de la poudre sèche de feuilles dans de l'acétone contenant 40 % en poids d'eau ;

ii. élimination des lipides par concentration de l'extrait sous pression réduite jusqu'à obtenir un concentré contenant environ 30 % de solides pour ainsi éliminer l'acétone puis dilution avec de l'eau pour obtenir une concentration de 15 % en solides, refroidissement de la suspension obtenue à environ 10° C pendant une heure avant d'éliminer par filtration les précipités lipidiques ;

iii. enrichissement de la phase organique par ajout d'une solution de sulfate d'ammonium à 30 % au filtrat aqueux suivi par une extraction liquide-liquide avec un mélange de méthyléthylcétone et d'acétone (entre 9:1 et 4:6) et concentration de la phase organique pour obtenir une concentration de 50 à 70 % en solides ;

iv. élimination des tannins par dilution du concentré dans un mélange d'eau et d'éthanol 50/50 en poids pour obtenir une concentration de 10 % en solides, addition d'une solution aqueuse d'un sel de plomb jusqu'à ce que la couleur soit passée de brun à ambre et filtration du précipité plomb-tannins pour donner un filtrat clair ;

v. élimination du plomb sous forme de son sulfate insoluble par concentration du filtrat pour garder une proportion maximum de 5 % en éthanol, ajout de sulfate d'ammonium jusqu'à une concentration d'environ 20 % suivi d'une extraction liquide-liquide avec un mélange de méthyléthylcétone et d'éthanol (entre 8:2 et 1:1) ; et

vi. séchage du produit final par concentration de la phase organique pour obtenir une concentration de 50 à 70 % en solides et séchage sous vide dans un four à 60-80° C pour isoler le produit final contenant moins de 5 % d'eau.

Depuis ces dix dernières années, de grands efforts ont été faits pour enrichir la part des composés actifs des extraits de *Ginkgo biloba* tout en réduisant par la même occasion la part des composés allergènes alkylphénoliques à longue chaîne tels que les acides ginkgoliques.

Il existe un certain nombre de demandes de brevets et de brevets décrivant des procédés permettant d'obtenir des extraits de feuilles de *Ginkgo biloba* enrichis en flavone-glycosides et terpène-lactones.

Par exemple, le brevet US 5,389,370 concerne des méthodes de préparation d'extraits concentrés en composés actifs. La stratégie employée dans ce cas est de mélanger la fraction riche en flavone-glycosides obtenue par extraction liquide-liquide avec du bilobalide et des ginkgolides isolés grâce à une séparation chromatographique sur colonne pour obtenir les compositions désirées qui contiennent typiquement 50 % de flavone-glycosides et 6 % de bilobalide, 50 % de flavone-glycosides et 7 % de ginkgolides ou 50 % de flavone-glycosides, 6 % de bilobalide et 7 % de ginkgolides. Le procédé décrit reprend les deux premières étapes du brevet US 5,399,348 cité précédemment (qui seront ici désignées comme les étapes 1 et 2) auxquelles sont ajoutées les étapes successives suivantes :

3. enrichissement des phases organiques : extraction liquide-liquide avec un mélange d'acétate d'éthyle et d'hexane (9:1), la phase aqueuse étant ré-extraite avec du *n*-butanol et la phase *n*-butanol obtenue concentrée à sec pour obtenir la fraction riche en flavone-glycosides ;

4. traitement au charbon actif : lavage de la phase acétate d'éthyle-hexane avec de l'eau, la phase acétate d'éthyle-hexane lavée étant ensuite traitée avec du charbon actif, filtrée et concentrée à sec ;

5. recristallisation : dissolution du résidu solide dans un mélange 50/50 en poids d'éthanol et d'eau, refroidissement pour cristalliser et filtration pour récupérer les ginkgolides ;

6. chromatographie sur colonne : concentration à sec du surnageant de l'étape 5 et séparation chromatographique sur colonne de gel de silice pour obtenir des fractions riches en bilobalide et en ginkgolides ; et

7. mélange des fractions : mélange de la fraction riche en flavone-glycosides de l'étape 3 avec les ginkgolides obtenus à l'étape 5 ou 6 et/ou avec le bilobalide obtenu à l'étape 6.

Selon une variante du procédé ci-dessus, les étapes 4 et 5 peuvent être remplacées par une chromatographie sur colonne.

Par ailleurs, le brevet US 6,030,621 décrit une méthode d'obtention d'extraits de *Ginkgo biloba* enrichi en flavone-glycosides et en terpène-lactones. La stratégie employée est analogue à celle du brevet US 5,389,370: la fraction riche en flavone-glycosides est mélangée avec les fractions riches en terpène-lactones, ces dernières étant obtenues par chromatographie sur colonne. Typiquement, les extraits préparés comprennent 47,2 % de flavone-glycosides et 6,3 % de terpène-lactones ou 70 % de flavone-glycosides et 10 % de terpène-lactones. Cette méthode comprend les étapes suivantes :

a) extraction de la poudre sèche de feuilles dans de l'éthanol contenant 50 % en poids d'eau ;

b) élimination des lipides par concentration de l'extrait sous pression réduite jusqu'à élimination de l'éthanol, dilution du concentré avec de l'eau, repos du mélange pendant 48 heures et filtration des précipités lipidiques ;

c) piégeage des composés actifs par passage du filtrat aqueux à travers une résine consistant en un mélange de résine XAD-4 (mélange de polymères poreux) et de polyamide ;

d) élution des composés actifs en utilisant des mélanges éthanol-eau successifs contenant 30, 60 et 90 % en poids d'éthanol ; et

e) combinaison de toutes fractions obtenues en d), évaporation de l'éthanol, lavage du concentré aqueux avec de l'hexane et évaporation à sec de la phase aqueuse lavée pour obtenir un extrait contenant 47,2 % de flavone-glycosides et 6,3 % de terpène-lactones.

Dans une variante du procédé ci-dessus, la fraction obtenue à partir du mélange éthanol-eau contenant 30 % d'éthanol est soumise à une chromatographie-éclair pour obtenir une fraction contenant au moins 80 % de terpène-lactones. De la même façon, les fractions obtenues à partir des mélanges éthanol-eau contenant 60 et 90 % d'éthanol sont combinées et soumises à une chromatographie-éclair pour obtenir une fraction contenant au moins 80 % de flavone-glycosides. La fraction contenant au moins 80 % de flavone-glycosides et la fraction contenant au moins 80 % de terpène-lactones sont mélangées dans différentes proportions et permettent d'obtenir des extraits contenant jusqu'à 70 % de flavone-glycosides et 10 % de terpène-lactones.

A cause des exigences croissantes en matière de sécurité sanitaire, il devient préférable d'utiliser des principes actifs de plus en plus purs. La tendance pour les extraits de *Ginkgo biloba* serait donc d'abandonner un jour les extraits comme l'EGb 761® au profit d'extraits encore plus riches en principes actifs. Typiquement, on pourrait viser des extraits contenant au moins 50 % en poids de flavone-glycosides et 12 % en poids de terpène-lactones, ou bien des extraits contenant au moins 30 % en poids de terpène-lactones et au moins 15 % en poids de flavone-glycosides. Les procédés des brevets US 5,389,370 ou 6,030,621 permettraient d'obtenir un tel extrait mais s'avèrent toutefois onéreux lorsqu'il s'agit de les utiliser à une échelle industrielle en raison principalement de la présence d'étapes de séparation par chromatographie.

La demanderesse vient de mettre au point une méthode permettant d'obtenir des extraits de feuilles de *Ginkgo biloba* répondant aux spécifications indiquées sans utiliser aucune étape de séparation par chromatographie.

L'invention a donc pour objet un procédé de préparation d'un extrait de feuilles de *Ginkgo biloba,* lequel comprend les étapes successives suivantes :

i. extraction de fragments secs de feuilles de *Ginkgo biloba* dans de l'éthanol contenant au maximum 20 % en poids d'eau ;

ii. concentration de l'extrait sous pression réduite en présence d'une solution aqueuse de chlorure de sodium et élimination de l'huile sombre du reste de la solution claire ;

iii. lavage de la solution aqueuse résiduelle par extraction liquide-liquide avec du n-hexane, du n-heptane ou du cyclohexane ;

iv. extraction liquide-liquide de la phase aqueuse lavée avec de l'acétate d'éthyle ;

v. lavage de la phase acétate d'éthyle obtenue à l'étape iv avec une solution de chlorure de sodium puis évaporation à sec de la phase acétate d'éthyle lavée.

Par fragments secs de feuilles de *Ginkgo biloba,* on entend dans la présente demande des fragments secs dont la taille des particules n'excède pas 5 mm.

De préférence selon l'invention, les fragments secs de feuilles de *Ginkgo biloba* seront sous forme de poudre sèche. Par poudre, on entend dans la présente demande une poudre dont la taille des particules n'excède pas 1 mm (et de préférence pas 0,5 mm).

Le procédé selon l'invention peut être appliqué :

- soit à des fragments (et de préférence à une poudre) de feuilles de *Ginkgo biloba* lyophilisées, auquel cas on obtiendra des extraits contenant au moins 50 % en poids de flavone-glycosides et 12 % en poids de terpène-lactones ;
- soit à des fragments (et de préférence à une poudre) de feuilles de *Ginkgo biloba* séchées, auquel cas on obtiendra des extraits - ci-après dénommés « extraits principaux » - contenant au moins 30 % en poids de terpène-lactones et au moins 15 % en poids de flavone-glycosides (ledit procédé permettant dans ce cas d'obtenir en outre en tant que produit annexe des extraits - ci-après dénommés « extraits annexes » - analogues à ceux décrits dans la demande de brevet PCT WO 96/33728, à savoir des extraits contenant environ 30 à 35% en poids de flavone-glycosides et environ 1% en poids de terpène-lactones).

Selon l'une des variantes de l'invention, le procédé décrit ci-dessus sera appliqué à des fragments (ou à une poudre) de feuilles de *Ginkgo biloba* lyophilisées et l'extrait obtenu sera de préférence tel qu'il contienne environ 52 % en poids de flavone-glycosides et environ 13 % en poids de terpène-lactones.

Selon la variante préférée de l'invention, le procédé décrit ci-dessus sera appliqué à des fragments (ou à une poudre) de feuilles de *Ginkgo biloba* séchées et l'extrait principal obtenu sera de préférence tel qu'il contienne de 34 à 46 % en poids de terpène-lactones et de 18 à 30 % en poids de flavone-glycosides. Plus préférentiellement, le procédé décrit ci-dessus sera appliqué à des fragments (ou à une poudre) de feuilles de *Ginkgo biloba* séchées et l'extrait principal obtenu sera tel qu'il contienne de 36 à 44 % en poids de terpène-lactones et de 20 à 28 % en poids de flavone-glycosides. En particulier, le procédé décrit ci-dessus sera appliqué à des fragments (ou à une poudre) de feuilles de *Ginkgo biloba* séchées et l'extrait principal obtenu contiendra environ 40 % en poids de terpène-lactones (dont environ 24,5 % en poids de ginkgolides A, B et C et environ 15,5 % en poids de bilobalide) et environ 24 % en poids de flavone-glycosides.

De préférence, l'extraction de l'étape i sera effectuée à l'aide d'éthanol contenant entre 10 et 20 % en poids d'eau, et plus préférentiellement entre 15 et 20 % en poids d'eau.

De préférence aussi, le mélange obtenu après concentration de l'extrait sous pression réduite en présence d'une solution aqueuse de chlorure de sodium lors de l'étape ii sera refroidi, par exemple à une température d'environ 10 °C, de préférence pendant une durée de 10 ou 15 minutes à 1 ou 2 heures, avant d'effectuer élimination de l'huile sombre du reste de la solution claire. Toujours de préférence, de la célite sera ajoutée au mélange obtenu après concentration de l'extrait sous pression réduite en présence d'une solution aqueuse de chlorure de sodium lors de l'étape ii et la totalité de l'éthanol présent dans ledit mélange sera évaporée avant de procéder au refroidissement dudit mélange.

De préférence également, le lavage de l'étape iii sera effectué avec du n-heptane.

Selon une variante particulièrement préférée du procédé de l'invention lorsque celui-ci est appliqué à des fragments (ou à une poudre) de feuilles de *Ginkgo biloba* séchées, le volume de la solution aqueuse résiduelle de l'étape iii et la quantité de chlorure de sodium dans la solution seront préalablement ajustés afin que, d'une part, la teneur en poids en solides hydrosolubles soit d'environ 8% par rapport au poids total de la solution, et d'autre part, la teneur en poids en chlorure de sodium soit de 16% par rapport au poids total de la solution. La teneur en solides hydrosolubles est évaluée par prélèvement d'un échantillon de solution éthanolique qui est évaporé pour éliminer l'éthanol puis extrait au dichlorométhane, la phase aqueuse résiduelle étant ensuite évaporée à sec et pesée ; la teneur en solides hydrosolubles peut alors être déduite de la masse de résidu solide obtenue.

En ce qui concerne l'étape iv, de la méthyléthylcétone contenant de 2 à 10% d'acétone ou d'éthanol en termes de volume pourrait éventuellement remplacer l'acétate d'éthyle.

En ce qui concerne les solutions aqueuses de chlorure de sodium employées aux étapes ii et v, elles auront de préférence une concentration d'au moins 10 % en poids de chlorure de sodium pour l'étape ii tandis qu'elles seront de préférence saturées en chlorure de sodium pour l'étape v.

Selon une variante préférée du procédé ci-dessus appliqué à des fragments (ou une poudre) de feuilles de *Ginkgo biloba* séchées, la phase aqueuse saline récupérée à l'issue de l'extraction liquide-liquide de l'étape iv pourra être retraitée par un procédé comportant les étapes suivantes :

a) extraction liquide-liquide à l'aide d'un mélange environ 1:1 d'acétate d'éthyle et d'éthanol ; et

b) évaporation à sec de la phase organique récupérée à l'issue de l'étape a) ;

c) dissolution du résidu obtenu à l'étape b) dans de l'éthanol absolu afin d'avoir une concentration d'environ 5% à 10% en poids de solides par rapport à la masse de la solution éthanolique ;

d) refroidissement du mélange à une température de préférence inférieure ou égale à 10 °C (par exemple à une température de 2 à 8 °C), de préférence pendant une durée de 30 minutes à 12 heures ;

e) filtration et évaporation de l'éthanol du filtrat récupéré pour donner un extrait sec.

Ce procédé additionnel permet de récupérer un extrait supplémentaire, lequel contient généralement environ 30 à 35% en poids de flavone-glycosides et environ 1% en poids de terpène-lactones.

Optionnellement, le procédé décrit ci-dessus pourra comporter, après les étapes i à v, l'étape vi suivante :

vi. mise en solution de l'extrait sec dans de l'éthanol et refroidissement de la solution à une température de préférence inférieure ou égale à 10 °C (par exemple à une température de 2 à 8 °C), filtration du sel éventuellement précipité et évaporation à sec de la solution résultante.

Dans ce cas, lorsque le procédé de l'invention sera utilisé à l'échelle industrielle, on préférera pour l'étape v simplement concentrer la phase organique jusqu'à obtenir une concentration d'environ 50 à 70% en poids de solides (et non l'évaporer à sec) et ajouter de l'éthanol pour avoir une composition contenant 5 à 20% en poids d'eau, 5 à 20% en poids de solides et le restant en éthanol avant d'effectuer l'étape vi. En procédant ainsi, on économisera une étape de séchage.

Les extraits principaux obtenus selon l'invention sont utilisables en pharmacie ou dans le domaine alimentaire (par exemple en tant qu'ingrédients de suppléments nutritionnels) car, d'une part, ils contiennent moins de 5 ppm d'alkylphénols (mesure effectuée par dosage HPLC) et, d'autre part, ils contiennent moins de 0,3 % de prodelphinidines (de préférence moins de 0,25 % et généralement entre 0,05 et 0,25 % de prodelphinidines). Par ailleurs, ils recèlent également peu d'impuretés lipophiles et peu de polysaccharides, de proanthocyanidines autres que les prodelphinidines et de protéines à haute masse moléculaire.

En particulier, du fait de leur faible teneur en prodelphinidines, les extraits principaux selon l'invention seront mieux adaptés à une administration aux patients par la voie intraveineuse. A titre de comparaison, un extrait commercial tel que l'EGb 761® peut contenir jusqu'à 1,5% de prodelphinidines.

En outre, les extraits principaux de l'invention seront de préférence tels que les pics correspondant au O-rhamnopyranosyl-4-O-D-(trans-p-coumaroyl-6''')glucopyranosyloxy-3-tétrahydroxy-3',4',5,7-flavonol (ou 3-(6'''-trans-p-coumaroyl)-glucorhamnoside de quercétine) O-rhamnopyranosyl-4-O-D-(trans-p-coumaroyl-6''')glucopyranosyloxy-3-trihydroxy-4',5,7-flavonol (ou 3-(6'''-trans-p-coumaroyl)-glucorhamnoside de kaempferol) représenteront ensemble environ 39% de la surface totale des pics du chromatogramme. En particulier, les extraits principaux de l'invention seront de préférence tels que le pic correspondant au O-rhamnopyranosyl-4-O-D-(trans-p-coumaroyl-6''')glucopyranosyloxy-3-tétrahydroxy-3',4',5,7-flavonol (ou 3-(6'''-trans-p-coumaroyl)-glucorhamnoside de quercétine) représentera environ 20% de la surface totale des pics du chromatogramme. De même, les extraits principaux de l'invention seront de préférence tels que le pic correspondant au O-rhamnopyranosyl-4-O-D-(trans-p-coumaroyl-6''')glucopyranosyloxy-3-trihydroxy-4',5,7-flavonol (ou 3-(6'''-trans-p-coumaroyl)-glucorhamnoside de kaempferol) représentera environ 19% de la surface totale des pics du chromatogramme.

Par ailleurs, les extraits annexes obtenus à l'aide du procédé selon l'invention contiendront moins de 0,5% de prodelphinidines (de préférence moins de 0,40 % et généralement entre 0,15 et 0,40% de prodelphinidines).

L'invention a de plus pour objet les extraits principaux susceptibles d'être obtenus par un procédé selon l'invention. Elle a également pour objet lesdits extraits principaux à titre de médicaments, les compositions pharmaceutiques comportant, à titre de principe actif, lesdits extraits principaux et l'utilisation desdits extraits principaux pour préparer des médicaments destinés à traiter des maladies / désordres choisis parmi les maladies /désordres suivants : les désordres vasculaires cérébraux et périphériques (comme les acouphènes d'origine vasculaire, l'athérosclérose, les ischémies, les thromboses, les symptômes en relation avec l'insuffisance veineuse, les manifestations veineuses inflammatoires aiguës et les symptômes en relation avec une crise hémorroïdaire), les maladies neurodégénératives (comme par exemple la maladie d'Alzheimer, la maladie de Parkinson, la chorée de Huntington ou la sclérose latérale amyotrophique) et le déficit pathologique cognitif et neurosensoriel chronique du sujet âgé (notamment la perte de mémoire observée chez le sujet âgé non atteint de maladie d'Alzheimer ou d'une autre démence). Elle a enfin pour objet l'utilisation desdits extraits principaux pour préparer des médicaments destinés à offrir une thérapie adjuvante pour traiter les maladies prolifératives (notamment le cancer).

Les compositions pharmaceutiques contenant un extrait de l'invention peuvent être sous forme solide comme, par exemple, les poudres, pilules, granules, comprimés, liposomes, gélules ou suppositoires. Les pilules, les comprimés ou les gélules peuvent être revêtus d'une substance capable de protéger la composition de l'action de l'acide gastrique ou des enzymes dans l'estomac du sujet pendant une période de temps suffisante pour permettre à cette composition de passer non digérée dans l'intestin grêle de ce dernier. L'extrait peut aussi être administré localement, par exemple à l'emplacement même d'une tumeur. L'extrait peut aussi être administré selon un processus de libération prolongée (par exemple en utilisant une composition à libération prolongée ou une pompe de perfusion). Les supports solides appropriés peuvent être, par exemple, le phosphate de calcium, le stéarate de magnésium, le carbonate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un extrait de l'invention peuvent également se présenter sous forme liquide comme, par exemple, des solutions, des émulsions, des suspensions ou une formulation à libération prolongée. Les supports liquides appropriés peuvent être, par exemple, l'eau, les solvants organiques tels que le glycérol ou les glycols tel que le polyéthylène glycol, de même que leurs mélanges, dans des proportions variées, dans l'eau.

L'administration d'un médicament selon l'invention pourra se faire par voie topique, orale, parentérale, par injection intramusculaire, etc.

La dose d'un extrait selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

Sans préjuger de l'avis du médecin traitant, et compte tenu de la teneur en principes actifs des extraits principaux selon l'invention, l'administration à un être humain d'une dose journalière de 5 à 150 mg, et de préférence d'une dose de 10 à 100 mg (en particulier une dose de 40 à 80 mg, par exemple de 50 à 70 mg) desdits extraits principaux pourra par exemple être envisagée.

Dans la présente demande, le terme "environ" fait référence à un intervalle autour de la valeur considérée. Tel qu'utilisé dans la présente demande, "environ X" signifie un intervalle de X moins 10% de X à X plus 10% de X, et de préférence un intervalle de X moins 5% de X à X plus 5% de X.

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples qui suivent sont présentés pour illustrer les procédés ci-dessus.

## EXEMPLES

### Exemple 1

100 g de feuilles lyophilisées de *Ginkgo biloba* pilées (taille des particules inférieure à 4 mm) sont extraites par deux fois, une fois avec 800 ml et une deuxième fois avec 600 ml d'un mélange éthanol-eau contenant 88 % en poids d'éthanol, chaque fois à 60° C et pendant 1 heure. Les extraits des deux étapes d'extraction sont filtrés et les restes de feuilles récupérés rincés avec 200 ml du même mélange de solvants. Les extraits combinés sont réduits par évaporation à un volume de 100 ml avec un contenu en produits solides d'environ 35 g. 350 ml d'une solution aqueuse de chlorure de sodium à 10 % en poids est ajouté au concentré éthanolique et l'évaporation est poursuivie sous pression réduite à 50° C pour éliminer le reste d'éthanol. La solution saline limpide orange clair est séparée de l'huile sombre par décantation à travers un tampon de coton sur un entonnoir filtrant puis par filtration sur célite avec succion. L'extrait en solution aqueuse saline est lavé avec deux portions de 150 ml de n-heptane avant d'être extrait par deux fois avec 150 ml d'acétate d'éthyle à chaque fois. Les phases acétate d'éthyle combinées sont lavées avec 50 ml d'une solution saturée en chlorure de sodium puis évaporées à sec sous pression réduite. L'extrait sec est redissous dans 60 ml d'éthanol absolu, laissé la nuit durant dans un réfrigérateur à 5° C, filtré pour éliminer le sel éventuellement précipité et le filtrat évaporé à sec pour donner 1,42 g d'extrait contenant 51,7 % de flavone-glycosides et 13 % de terpène-lactones (6,55 % de bilobalide).

### Exemple 2

100 g de feuilles de *Ginkgo biloba* obtenues après séchage dans une étuve rotative industrielle et pilées (taille des particules inférieures à 4 mm ; teneur de 1,8% en poids de flavone glycosides et de 0,12% en poids de ginkgolides) sont extraites par deux fois, une fois avec 800 ml et une deuxième fois avec 600 ml d'un mélange éthanol-eau contenant 80% en poids d'éthanol, chaque fois à 60 °C et pendant 1 heure. Les extraits des deux étapes d'extraction sont filtrés et les restes de feuilles récupérés rincés avec 200 ml du même mélange de solvants. Les extraits combinés sont réduits par évaporation à un volume de 800 ml avec un contenu en produits solides d'environ 33 g. 60 g de chlorure de sodium, 100 ml d'eau et 22 g de célite sont ajoutés au concentré éthanolique et l'évaporation est poursuivie sous pression réduite à 50 °C pour éliminer le reste d'éthanol. Le concentré aqueux est refroidi à 10 °C pendant une heure et la solution saline limpide orange clair est séparée de l'huile sombre par décantation à travers un tampon de coton sur un entonnoir filtrant puis par filtration avec succion sur fritté recouvert d'un lit de célite. L'extrait en solution aqueuse saline (- 300 ml) est lavé avec deux portions de 125 ml de n-heptane avant d'être extrait par deux fois avec 125 ml d'acétate d'éthyle à chaque fois. Les phases acétate d'éthyle combinées sont lavées avec 50 ml d'une solution saturée en chlorure de sodium puis évaporées à sec sous pression réduite. L'extrait sec est redissous dans 23 ml d'éthanol absolu, laissé la nuit durant dans un réfrigérateur à 4 °C, filtré pour éliminer le sel éventuellement précipité et le filtrat évaporé à sec pour donner 1,06 g d'extrait contenant 23,6 % de flavone-glycosides, 39,2 % de terpène-lactones (dont 24,6 % de ginkgolides A, B et C et 14,5 % de bilobalide) et environ 0,14% de prodelphinidines.

### Exemple 3

La phase aqueuse saline qui est récupérée après les étapes successives de lavage avec du *n*-heptane et d'extraction avec de l'acétate d'éthyle du procédé de préparation de l'extrait de l'exemple 2 est soumise à une extraction liquide-liquide à l'aide de deux portions de 125 ml d'un mélange acétate d'éthyle-éthanol 1:1. La phase organique récupérée est évaporée à sec. L'extrait sec est repris dans de l'éthanol absolu de sorte à avoir une concentration de 5% en poids d'extrait sec par rapport au poids total de la solution. Le mélange est laissé la nuit durant dans un réfrigérateur à 4 °C avant d'être filtré sur papier (on rince les solides avec de l'éthanol absolu à 4 °C). Après évaporation à sec, on obtient un extrait contenant 31,88% de flavone-glycosides et 0,67% de terpènes (dont 0,50% de ginkgolides A, B et C et 0,17 % de bilobalide) et environ 0,21% de prodelphinidines.

### Caractérisation des extraits selon l'invention

### A) HPLC :

Les extraits selon l'invention peuvent être caractérisés à l'aide de la méthode de Chromatographie Liquide Haute Performance (HPLC) avec élution par gradients décrite ci-après.

*Matériels*

- Chromatographe en phase liquide et équipement adapté aux conditions spécifiées ci-après
- Verrerie de laboratoire
- Balance de précision au $10^e$ de mg.

*Réactifs*

- Eau pure (qualité HPLC)
- Acétonitrile (qualité HPLC)
- Isopropanol (qualité HPLC)
- Acide citrique (qualité HPLC)
- Méthanol R
- Extrait de *Ginkgo biloba* standard de référence

*Procédure*

Une solution de l'extrait à tester est préparée par dissolution de 200 mg d'extrait à analyser dans 10 ml de méthanol. Parallèlement, 200 mg d'extrait de *Ginkgo biloba* standard de référence (EGb 761®) sont dissous dans 10 ml de méthanol et constituent la solution de référence.

Les conditions de chromatographie suivantes sont employées :

- Type de gradient : linéaire de 0% à 100% de phase mobile secondaire en 15 min puis 5 min avec la phase mobile secondaire. Rééquilibrer la colonne pendant 10 min avec la phase mobile primaire avant l'injection suivante.
- Phase mobile primaire :

  eau purifiée        1000 ml
  acétonitrile        200 ml
  isopropanol         30 ml
  acide citrique       4,92 g

- Phase mobile secondaire :

  eau purifiée        1000 ml
  acétonitrile        470 ml
  isopropanol         50 ml
  acide citrique       6,08 g

- Débit : 1,5 ml/min
- Détection : UV 360 nm
- Colonne : Acier inoxydable, 15 cm, diamètre 4,6 mm; remplie de gel de silice pour chromatographie R Nucleosil 5C18 Macherey-Nagel ou équivalent (5 $\mu$m).
- Volume injecté : 5 $\mu$l
- Température de colonne : 20 $\pm$ 2 °C

*Résultats*

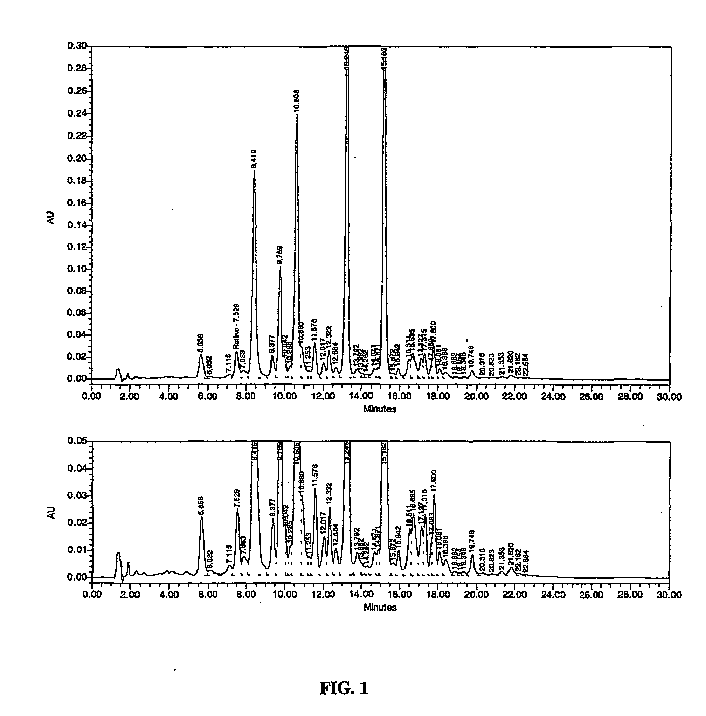

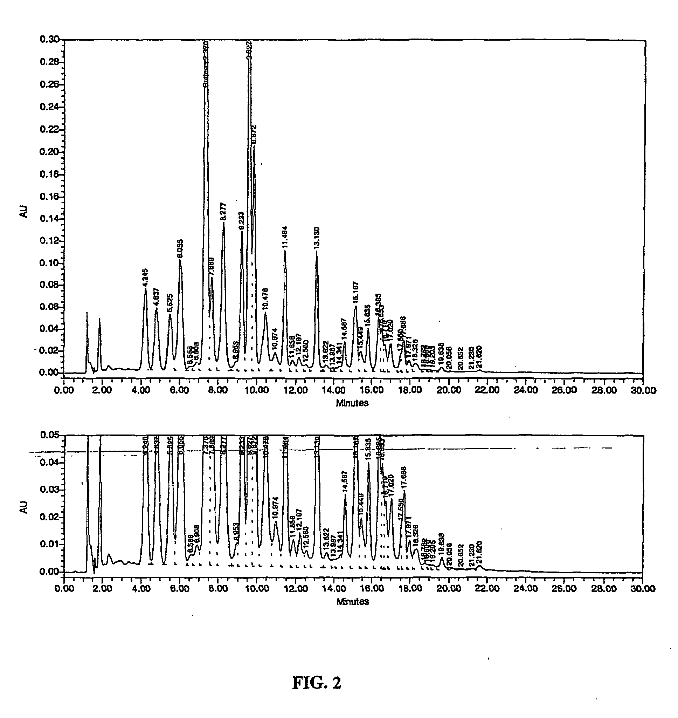

Le chromatogramme obtenu pour l'extrait obtenu à l'exemple 2 est reproduit en **Figure 1** et celui pour l'extrait obtenu à l'exemple 3 en **Figure 2.**

Les intégrations déterminées pour les différents pics sont reproduites dans les tableaux **I** (extrait de l'exemple 2) et **II** (extrait de l'exemple 3) ci-après. Les pics majeurs ayant des temps de rétention d'environ 13 et 15 minutes dans les conditions décrites ci-dessus correspondent respectivement au O-rhamnopyranosyl-4-O-D-(trans-p-coumaroyl-6''')glucopyranosyloxy-3-tétrahydroxy-3',4',5,7-flavonol (ou 3-(6'''-trans-p-coumaroyl)-glucorhamnoside de quercétine) et au O-rhamnopyranosyl-4-O-D-(trans-p-coumaroyl-6''')glucopyranosyloxy-3-trihydroxy-4',5,7-flavonol (ou 3-(6'''-trans-p-coumaroyl)-glucorhamnoside de kaempferol).

Tableau I

| N° du pic | T.R. (min) | Type intégration | Surface | % surface |
|---|---|---|---|---|
| 1 | 5,66 | BV | 323169,34 | 1,63 |
| 2 | 6,09 | VV | 40920,21 | 0,21 |
| 3 | 7,12 | VV | 69832,25 | 0,35 |
| 4 | 7,53 | VV | 335574,32 | 1,69 |
| 5 | 7,86 | VV | 119797,71 | 0,60 |
| 6 | 8,42 | VV | 2633527,95 | 13,25 |
| 7 | 9,38 | VV | 272916,32 | 1,37 |
| 8 | 9,76 | VV | 1226466,81 | 6,17 |
| 9 | 10,04 | VV | 72124,84 | 0,36 |
| 10 | 10,29 | Vv | 97259,69 | 0,49 |

Tableau I   (suite)

| N° du pic | T.R. (min) | Type intégration | Surface | % surface |
|---|---|---|---|---|
| 11 | 10,61 | vv | 2967432,21 | 14,93 |
| 12 | 10,88 | vV | 343065,16 | 1,73 |
| 13 | 11,25 | VV | 61980,66 | 0,31 |
| 14 | 11,58 | VV | 405705,79 | 2,04 |
| 15 | 12,02 | VV | 193158,96 | 0,97 |
| 16 | 12,32 | VV | 295426,85 | 1,49 |
| 17 | 12,66 | VV | 139571,25 | 0,70 |
| 18 | 13,25 | VV | 39600621,99 | 19,93 |
| 19 | 13,79 | Vv | 145778,77 | 0,73 |
| 20 | 13,99 | vV | 38952,25 | 0,20 |
| 21 | 14,26 | VV | 39634,91 | 0,20 |
| 22 | 14,67 | VV | 119350,40 | 0,60 |
| 23 | 14,87 | VV | 85122,51 | 0,43 |
| 24 | 15,18 | VV | 3789171,34 | 19,07 |
| 25 | 15,67 | VV | 60575,37 | 0,30 |
| 26 | 15,94 | VV | 118616,10 | 0,60 |
| 27 | 16,51 | VV | 208977,01 | 1,05 |
| 28 | 16,69 | VV | 345205,90 | 1,74 |
| 29 | 17,13 | VV | 206765,44 | 1,04 |
| 30 | 17,32 | VV | 241246,27 | 1,21 |
| 31 | 17,68 | Vv | 95087,16 | 0,48 |
| 32 | 17,80 | vV | 311413,97 | 1,57 |
| 33 | 18,08 | VV | 108167,40 | 0,54 |
| 34 | 18,40 | VV | 103158,38 | 0,52 |
| 35 | 18,88 | VV | 19884,37 | 0,10 |
| 36 | 19,15 | VV | 9701,13 | 0,05 |
| 37 | 19,35 | VV | 18389,23 | 0,09 |
| 38 | 19,75 | VV | 109237,26 | 0,55 |
| 39 | 20,42 | VV | 21754,52 | 0,11 |
| 40 | 20,82 | VV | 13348,93 | 0,07 |
| 41 | 21,35 | VV | 26491,23 | 0,13 |
| 42 | 21,82 | VV | 55867,45 | 0,28 |
| 43 | 22,18 | VV | 14634,07 | 0,07 |
| 44 | 22,58 | VB | 7095,01 | 0,04 |
| TOTAL | | | 19872178,50 | |

Tableau II

| N° du pic | T.R. (min) | Type intégration | Surface | % surface |
|---|---|---|---|---|
| 1 | 4,25 | BV | 1082912,49 | 3,74 |
| 2 | 4,84 | VV | 853793,45 | 2,95 |
| 3 | 5,52 | VV | 767098,18 | 2,65 |
| 4 | 6,06 | VV | 1634400,29 | 5,68 |
| 5 | 6,59 | VV | 53647,35 | 0,19 |
| 6 | 6,91 | VV | 127266,30 | 0,44 |
| 7 | 7,37 | VV | 5275614,83 | 18,22 |
| 8 | 7,69 | VV | 1062334,77 | 3,67 |
| 9 | 8,28 | VV | 1918663,93 | 6,63 |
| 10 | 8,95 | Vv | 103523,93 | 0,36 |
| 11 | 9,23 | vV | 1390204,37 | 4,80 |
| 12 | 9,63 | VV | 3866788,04 | 13,35 |
| 13 | 9,87 | VV | 2102182,30 | 7,26 |
| 14 | 10,48 | VV | 968935,87 | 3,35 |
| 15 | 10,97 | VV | 314304,16 | 1,0 |
| 16 | 11,46 | VV | 1167341,28 | 4,03 |
| 17 | 11,86 | VV | 143179,61 | 0,49 |
| 18 | 12,20 | VV | 193515,76 | 0,67 |
| 19 | 12,56 | Vv | 91823,99 | 0,32 |
| 20 | 13,13 | vV | 1197144,99 | 4,13 |
| 21 | 13,62 | VV | 94540,18 | 0,33 |
| 22 | 13,99 | VV | 33698,24 | 0,12 |
| 23 | 14,34 | VV | 78763,83 | 0,27 |
| 24 | 14,59 | VV | 334542,58 | 1,16 |
| 25 | 15,19 | VV | 944041,64 | 3,26 |
| 26 | 15,45 | VV | 244838,93 | 0,85 |
| 27 | 15,84 | VV | 473019,80 | 1,51 |
| 28 | 16,39 | VV | 569694,64 | 1,97 |
| 29 | 16,55 | Vv | 323620,29 | 1,12 |
| 30 | 16,72 | vV | 255311,37 | 0,88 |
| 31 | 17,02 | VV | 370249,91 | 1,28 |
| 32 | 17,55 | Vv | 106207,91 | 0,37 |
| 33 | 17,69 | vV | 338307,55 | 1,17 |
| 34 | 17,97 | VV | 134115,60 | 0,46 |
| 35 | 18,33 | VV | 148609,32 | 0,51 |
| 36 | 18,78 | Vv | 31564,17 | 0,11 |
| 37 | 18,91 | vV | 17047,61 | 0,06 |

Tableau II   (suite)

| N° du pic | T.R. (min) | Type intégration | Surface | % surface |
|---|---|---|---|---|
| 38 | 19,20 | VV | 25147,79 | 0,09 |
| 39 | 19,64 | VV | 67214,23 | 0,23 |
| 40 | 20,06 | VV | 16329,48 | 0,06 |
| 41 | 20,65 | VV | 11266,06 | 0,04 |
| 42 | 21,23 | VV | 10502,29 | 0,04 |
| 43 | 21,62 | VV | 40624,23 | 0,14 |
| TOTAL | | | 28956906,57 | |

**B) Détermination de la teneur en prodelphinidines :**

La teneur en prodelphinidines des extraits de l'invention est évaluée comme exposé ci-après.

Environ 50 mg d'extrait sont dissous dans 25 ml d'un mélange d'isopropanol et de solution 3N d'acide chlorhydrique (5/1 v/v). 5 ml du mélange obtenu sont transférés dans une fiole de 10 ml fermée par un bouchon étanche et la fiole est ensuite immergée dans l'eau bouillante pendant 45 minutes. Après refroidisssement à 20 °C, on amène le volume à 10 ml en ajoutant du mélange d'isopropanol et de solution 3N d'acide chlorhydrique. L'absorbance est mesurée à 556 nm et le pourcentage de prodelphinidines est obtenu en effectuant le calcul suivant :

$$\% \text{ prodelphinidines} = A \times 86{,}206897 / B$$

A : absorbance à 556 nm
B : mg d'échantillon

Pour diminuer l'incertitude quant au résultat, l'expérience est répétée 3 fois et une moyenne est effectuée sur les résultats obtenus.

**Propriétés pharmacologiques des extraits selon l'invention**

Afin de démontrer les propriétés pharmacologiques d'extraits selon l'invention, les tests suivants peuvent être menés.

1) Prolifération cellulaire

*Lignées de cellules*

Les lignées de cellules de cancer du sein MDA-231 ont été acquises au Lombardi Cancer Center (Georgetown University Medical Center). Ces lignées de cellules sont cultivées dans des récipients de culture en polystyrène (Corning) et multipliées dans le milieu Eagle modifié de Dulbecco (DMEM) supplémenté avec 10 % de sérum foetal bovin (FBS).

*Tests de liaison*

Les cellules MDA-231 sont dispersées par trituration dans 5 ml de solution saline de tampon phosphate (SSTP) puis centrifugées à 500 g pendant 15 minutes. Les cellules centrifugées sont resuspendues dans une SSTP et testées pour mesurer leur teneur en protéines. Des tests [3H]PK 11195 peuvent être effectués comme décrit dans Papadopoulos et coll., *J. Biol. Chem.* (1990), **265,** 3772-3779 et Hardwick et coll., *Cancer Res.* (1999), **59**, 831-842. Le [N-méthyl-3H]PK 11195 ou (1-(2-chlorophényl)-N-méthyl-N-(1-méthyl-propyl)-3-isoquinolinecarboxamide est obtenu auprès de Du Pont-New England Nuclear (Wilmington, DE, Etats-Unis) et le PK 11195 auprès de Research Biochemicals Incorporated (Natick, MA, Etats-Unis). Les résultats obtenus sont analysés à l'aide du programme LIGAND (Munson et Robard, *Anal. Biochem*. (1976), **72**, 248-254.

*Analyse ARN de Northern*

L'expression de l'ARNm de récepteurs benzodiazépine de type périphérique (PBR) dans des cellules MDA-231 traitées par l'extrait de l'exemple 1 ou de l'exemple 2 ou de l'EGb 761® (référence) peut être évaluée à l'aide d'une analyse de Northem comme décrit dans Hardwick et coll., *Cancer Res.* (1999), **59**, 831-842. L'ARN cellulaire total a été isolé à l'aide du réactif RNAzol B (TEL-TEST, Inc., Friendswood, TX, Etats-Unis) et de chloroforme. 20 µg d'ARN total est passé sur du gel à 1 % d'agarose et transféré durant la nuit sur des membranes de nylon (S&S Nytran, Scheicher & Schuell, Keene, NH, Etats-Unis). Un fragment d'ADNc humain de PBR d'une longueur de 0,2 kb (dérivé du vecteur plasmide pCMV5-PBR contenant la séquence complète du PBR humain) est marqué à l'aide de [$\alpha$-$^{32}$P] dCTP à l'aide d'un système aléatoire d'identification de *primers* ADN (Life Technologies, Gaithersburg, MD, Etats-Unis). Les conditions d'hybridation utilisées sont décrites dans la référence citée précédemment. L'autoradiographie est effectuée en exposant les *blots* à un film X-OMAT AR (Kodak, Rochester, NY, Etats-Unis) à -70° C pendant 4-48 heures. La quantification d'ARNm de PBR est faite à l'aide du logiciel SigmaGel (Jandel Scientific, San Rafel, CA, Etats-Unis).

*Prolifération cellulaire*

Des cellules MDA-231 sont placées sur des plaques à 96 puits (Corning, NJ, Etats-Unis) à une concentration d'environ 10 000 cellules par puits (24 h d'incubation) ou 5 000 cellules par puits (48 h d'incubation) dans du DMEM supplémenté avec 0,1 % de FBS. Les cellules sont ensuite incubées dans du FBS à 10 % avec des concentrations variées en composé de l'exemple ou de composé référence (EGb 761®) pendant les périodes indiquées ci-dessus. Les différences en terme de prolifération cellulaire sont analysées par mesure de la quantité de 5-bromo-2'-déoxyuridine (BrdU) incorporée, laquelle est déterminée en utilisant le matériel BrdU ELISA (Boehringer Mannheim, Indianapolis, IN, Etats-Unis). L'incorporation de BrdU est mesurée à la longueur d'onde de 450 nm (référence : 690 nm).

2) <u>Neurotoxicité induite par le glutamate</u>

Un extrait selon l'invention peut être testé comme suit : des neurones de cerveau de rat, sont traités ou non avec des concentrations croissantes d'extrait de l'exemple 1 ou de l'exemple 2 ci-dessus puis, 30 minutes après, sont exposés à du glutamate à une concentration de 250 µM. La dose inhibant à 50 % la toxicité du glutamate (désignée par IC$_{50}$) peut être alors être évaluée puis comparée à celle obtenue pour un extrait comme l'EGb 761®, moins riche en flavone-glycosides et terpène-lactones (à savoir 100 µg d'EGb 761® par ml).

3) <u>Toxicité cellulaire</u>

Les effets d'un extrait selon l'invention sur la viabilité des cellules peuvent être étudiés sur une lignée de cellules HT 22 (cellules hippocampales de souris). La viabilité des cellules est estimée par détermination de la quantité de LDH libérée par des cellules traitées par des doses croissantes de l'extrait de l'exemple 1 ou de l'exemple 2 ci-dessus et par le test d'exclusion au bleu Trypan.

*Lignées de cellules*

La lignée de cellules HT-22 provient du Salk Institute for Biological Research (La Jolla, CA, Etats-Unis). Cette lignée de cellules est maintenue à 37° C dans le milieu Eagle modifié de Dulbecco (DMEM) supplémenté avec 10 % de sérum foetal bovin (FBS), ci-après appelé milieu complet.

*Protocole du test*

Des cellules HT-22 sont placées sur une plaque à 96 puits à une concentration de 5.10$^3$ cellules par puits dans le milieu complet. 24 heures après, l'extrait de l'exemple est solubilisé dans du DMEM et ajouté à la concentration de 10, 25, 50, 100, 250, 500 et 1000 µg/ml ; l'extrait est alors laissé en contact pendant 24 ou 48 heures. La mesure de LDH se fait à l'aide d'un coffret de dosage Promega selon les instructions données par le fabricant. Les absorbances sont lues à 470 nm. La libération maximale de LDH est obtenue après lyse complète des cellules à l'aide de Triton X-100.
Pour les mesures de viabilité estimée par le test d'exclusion au bleu Trypan, les cellules sont placées dans des plaques 6 puits et l'extrait de l'exemple 1 ou de l'exemple 2 est ajouté à la concentration de 100 µg/ml pendant 24 heures.
Les cellules sont ensuite lavées avec une solution tampon phosphate exempte de calcium et de magnésium et exposées à de la trypsine pendant 5 minutes à 37° C. La réaction est stoppée avec du milieu complet et 0,04 % de bleu de trypan est ajouté à la suspension cellulaire. Le nombre de cellules qui excluent le colorant (i.e. les cellules vivantes) est déterminé à l'aide d'un hématocytomètre. Le pourcentage de viabilité des cellules est calculé comme suit : nombre de cellules viables (non colorées) / nombre total de cellules (colorées et non colorées) x 100.

**Revendications**

1. Procédé de préparation d'un extrait de feuilles de *Ginkgo biloba*, lequel comprend les étapes successives suivantes :

   i. extraction de fragments secs de feuilles de *Ginkgo biloba* dans de l'éthanol contenant au maximum 20 % en poids d'eau ;

   ii. concentration de l'extrait sous pression réduite en présence d'une solution aqueuse de chlorure de sodium et élimination de l'huile sombre du reste de la solution claire ;

   iii. lavage de la solution aqueuse résiduelle par extraction liquide-liquide avec du n-hexane, du n-heptane ou du cyclohexane ;

   iv. extraction liquide-liquide de la phase aqueuse lavée avec de l'acétate d'éthyle ;

   v. lavage de la phase acétate d'éthyle obtenue à l'étape iv avec une solution de chlorure de sodium puis évaporation à sec de la phase acétate d'éthyle lavée.

2. Procédé selon la revendication 1, **caractérisé en ce qu'**il ne comporte aucune étape chromatographique.

3. Procédé selon la revendication 1 ou 2 dans lequel les fragments secs de feuilles de *Ginkgo biloba* de l'étape i sont remplacés par une poudre sèche de feuilles de *Ginkgo biloba*.

4. Procédé selon l'une des revendications 1 à 3, **caractérisé en ce que** l'extraction de l'étape i est effectuée à l'aide d'éthanol contenant de 10 à 20 % en poids d'eau.

5. Procédé selon l'une des revendications 1 à 4, **caractérisé en ce que** le lavage de l'étape iii est effectué avec du n-heptane.

6. Procédé selon l'une des revendications 1 à 5, **caractérisé en ce que** les solutions aqueuses de chlorure de sodium employées aux étapes ii et v ont une concentration d'au moins 10 % en poids de chlorure de sodium.

7. Procédé selon l'une des revendications 1 à 6 comprenant en outre, après les étapes i à v, l'étape vi suivante :

   vi. mise en solution de l'extrait sec dans de l'éthanol et refroidissement de la solution, filtration du sel éventuellement précipité et évaporation à sec de la solution résultante.

8. Extrait de feuilles de *Ginkgo biloba* susceptible d'être obtenu par un procédé de préparation selon l'une des revendications 1 à 7.

9. Extrait de feuilles de *Ginkgo biloba* selon la revendication 8, **caractérisé en ce que** le procédé est effectué à partir de fragments secs de feuilles séchées de *Ginkgo biloba*.

10. Extrait de feuilles de *Ginkgo biloba* selon la revendication 9, **caractérisé en ce qu'**il contient de 34 à 46 % en poids de terpène-lactones et de 18 à 30 % en poids de flavone-glycosides.

11. Extrait de feuilles de *Ginkgo biloba* selon la revendication 8, **caractérisé en ce que** le procédé est effectué à partir de fragments secs de feuilles lyophilisées de *Ginkgo biloba*.

12. Extrait de feuilles de *Ginkgo biloba* selon la revendication 11, **caractérisé en ce qu'**il contient environ 52 % en poids de flavone-glycosides et environ 13 % en poids de terpène-lactones.

**13.** A titre de médicament, un extrait selon l'une des revendications 8 à 12.

**14.** Composition pharmaceutique comportant, à titre de principe actif, un extrait selon l'une des revendications 8 à 12.

**15.** Utilisation d'un extrait selon l'une des revendications 8 à 12 pour préparer un médicament destiné à traiter des maladies / désordres choisis parmi les maladies / désordres suivants : les désordres vasculaires cérébraux et périphériques et les maladies neurodégénératives.

**16.** Utilisation d'un extrait selon l'une des revendications 8 à 12 pour préparer des médicaments destinés à offrir une thérapie adjuvante pour traiter les maladies prolifératives.

**Claims**

**1.** Process for the preparation of an extract of *Ginkgo biloba* leaves, which comprises the following successive stages:

> i. extraction of the dried fragments of *Ginkgo biloba* leaves in ethanol containing a maximum of 20 % by weight of water;

> ii. concentration of the extract under reduced pressure in the presence of an aqueous solution of sodium chloride and elimination of the dark oil from the remainder of the clear solution;

> iii. washing of the residual aqueous solution by liquid-liquid extraction with n-hexane, n-heptane or cyclohexane;

> iv. liquid-liquid extraction of the aqueous phase washed with ethyl acetate;

> v. washing of the ethyl acetate phase obtained in stage iv with a sodium chloride solution then evaporation to dryness of the washed ethyl acetate phase.

**2.** Process according to claim 1, **characterized in that** it includes no chromatographic stage.

**3.** Process according to claim 1 or 2, in which the dry fragments of *Ginkgo biloba* leaves of stage i are replaced by a dry powder of *Ginkgo biloba* leaves.

**4.** Process according to one of claims 1 to 3, **characterized in that** the extraction of stage i is carried out using ethanol containing from 10 to 20 % by weight of water.

**5.** Process according to one of claims 1 to 4, **characterized in that** the washing of stage iii is carried out with n-heptane.

**6.** Process according to one of claims 1 to 5, **characterized in that** the aqueous solutions of sodium chloride used in stages ii and v have a concentration of at least 10 % by weight of sodium chloride.

**7.** Process according to one of claims 1 to 6 comprising moreover, after stages i to v, the following stage vi:

> vi. solubilization of the dry extract in ethanol and cooling down of the solution, filtration of the optionally precipitated salt and evaporation to dryness of the resultant solution.

**8.** Extract of *Ginkgo biloba* leaves which can be obtained by a preparation process according to one of claims 1 to 7.

**9.** Extract of *Ginkgo biloba* leaves according to claim 8, **characterized in that** the process is carried out starting with dried fragments of dried *Ginkgo biloba* leaves.

**10.** Extract of *Ginkgo biloba* leaves according to claim 9, **characterized in that** it contains from 34 to 46 % by weight of terpene-lactones and from 18 to 30 % by weight of flavone-glycosides.

**11.** Extract of *Ginkgo biloba* leaves according to claim 8, **characterized in that** the process is carried out starting with dried fragments of lyophilized *Ginkgo biloba* leaves.

12. Extract of *Ginkgo biloba* leaves according to claim 11, **characterized in that** it contains approximately 52 % by weight of flavone-glycosides and approximately 13 % by weight of terpene-lactones.

13. As a medicament, an extract according to one of claims 8 to 12.

14. Pharmaceutical composition comprising an extract according to one of claims 8 to 12 as active ingredient.

15. Use of an extract according to one of claims 8 to 12 for preparing a medicament intended to treat diseases / disorders chosen from the following diseases / disorders: cerebral and peripheral vascular disorders and neurodegenerative diseases.

16. Use of an extract according to one of claims 8 to 12 for preparing medicaments intended to offer an adjuvant therapy for treating proliferative diseases.


**Patentansprüche**

1. Verfahren zur Herstellung eines Extrakts von Blättern von *Ginkgo biloba*, das die folgenden aufeinanderfolgenden Schritte umfaßt:

    i. Extraktion von trockenen Fragmenten von *Ginkgo-biloba*-Blättern in Ethanol, das maximal 20 Gew. -% Wasser enthält;
    ii. Konzentration des Extrakts unter vermindertem Druck in Gegenwart einer wässrigen Natriumchloridlösung und Entfernung des dunklen Öls aus dem Rest der hellen Lösung;
    iii. Waschen der wässrigen Restlösung durch Flüssig-Flüssig-Extraktion mit n-Hexan, n-Heptan oder Cyclohexan;
    iv. Flüssig-Flüssig-Extraktion der gewaschenen wässrigen Phase mit Ethylacetat;
    v. Waschen der im Schritt iv erhaltenen Ethylacetatphase mit einer Natriumchloridlösung und dann Eindampfen der gewaschenen Ethylacetatphase zur Trockene.

2. Verfahren nach Anspruch 1, **dadurch gekennzeichnet, daß** es keinen Chromatographieschritt umfaßt.

3. Verfahren nach Anspruch 1 oder 2, bei dem die trockenen Fragmente von Ginkgo-biloba-Blättern des Schritts i durch ein trockenes Pulver von *Ginkgo-biloba*-Blättern ersetzt wird.

4. Verfahren nach einem der Ansprüche 1 bis 3, **dadurch gekennzeichnet, daß** die Extraktion des Schritts i mit Hilfe von 10 bis 20 Gew.-% Wasser enthaltendem Ethanol durchgeführt wird.

5. Verfahren nach einem der Ansprüche 1 bis 4, **dadurch gekennzeichnet, daß** das Waschen des Schritts iii mit n-Heptan durchgeführt wird.

6. Verfahren nach einem der Ansprüche 1 bis 5, **dadurch gekennzeichnet, daß** die in den Schritten ii und v verwendeten wässrigen Natriumchloridlösungen eine Konzentration von mindestens 10 Gew.-% Natriumchlorid haben.

7. Verfahren nach einem der Ansprüche 1 bis 6, das außerdem nach den Schritten i bis v den folgenden Schritt vi umfaßt:

    vi. Lösung des trockenen Extrakts in Ethanol und Kühlen der Lösung, Filtration des ggf. ausgefällten Salzes und Eindampfen der sich ergebenden Lösung zur Trockene.

8. Extrakt von Blättern von *Ginkgo biloba*, der mit Hilfe eines Herstellungsverfahrens nach einem der Ansprüche 1 bis 7 erhältlich ist.

9. Extrakt von Blättern von *Ginkgo biloba* nach Anspruch 8, **dadurch gekennzeichnet, daß** das Verfahren an trockenen Fragmenten von getrockneten *Ginkgo-biloba*-Blättern vorgenommen wird.

**10.** Extrakt von Blättern von *Ginkgo biloba* nach Anspruch 9, **dadurch gekennzeichnet, daß** er 34 bis 46 Gew.-% Terpen-Lactone und 18 bis 30 Gew.-% Flavon-Glycoside enthält.

**11.** Extrakt von Blättern von *Ginkgo biloba* nach Anspruch 8, **dadurch gekennzeichnet, daß** das Verfahren an trockenen Fragmenten von lyophilisierten *Ginkgo-biloba*-Blättern vorgenommen wird.

**12.** Extrakt von Blättern von *Ginkgo biloba* nach Anspruch 11, **dadurch gekennzeichnet, daß** er etwa 52 Gew.-% Flavon-Glycoside und etwa 13 Gew.-% Terpen-Lactone enthält.

**13.** Extrakt nach einem der Ansprüche 8 bis 12 in Form eines Medikaments.

**14.** Pharmazeutische Zusammensetzung, die als Wirkstoff einen Extrakt nach einem der Ansprüche 8 bis 12 enthält.

**15.** Verwendung eines Extrakts nach einem der Ansprüche 8 bis 12 zur Herstellung eines Medikaments, das für die Behandlung von Krankenheiten bzw. Störungen bestimmt ist, die aus den folgenden Krankheiten bzw. Störungen ausgewählt sind: zerebralvaskuläre und periphere Störungen und neurodegenerative Krankheiten.

**16.** Verwendung eines Extrakts nach einem der Ansprüche 8 bis 12 zur Herstellung von Medikamenten, die für die unterstützende Therapie bei der Behandlung von proliferativen Krankheiten bestimmt sind.

**FIG. 1**

**FIG. 2**